(19) **Europäisches Patentamt / European Patent Office / Office européen des brevets**

(11) **EP 3 431 514 A1**

(12) **EUROPEAN PATENT APPLICATION**
published in accordance with Art. 153(4) EPC

(43) Date of publication:
**23.01.2019 Bulletin 2019/04**

(21) Application number: **17766866.2**

(22) Date of filing: **17.03.2017**

(51) Int Cl.:
*C08F 2/44* (2006.01)   *B29C 67/00* (2017.01)
*B33Y 10/00* (2015.01)   *B33Y 30/00* (2015.01)
*B33Y 70/00* (2015.01)

(86) International application number:
**PCT/JP2017/011009**

(87) International publication number:
**WO 2017/159871 (21.09.2017 Gazette 2017/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **18.03.2016 JP 2016055155**

(71) Applicant: **Ricoh Company, Ltd.**
**Tokyo 143-8555 (JP)**

(72) Inventors:
• **IWATA, Hiroshi**
**Tokyo 143-8555 (JP)**
• **NIIMI, Tatsuya**
**Tokyo 143-8555 (JP)**
• **NORIKANE, Yoshihiro**
**Tokyo 143-8555 (JP)**
• **MATSUMURA, Takashi**
**Tokyo 143-8555 (JP)**
• **NAITO, Hiroyuki**
**Tokyo 143-8555 (JP)**

(74) Representative: **Lamb, Martin John Carstairs**
**Marks & Clerk LLP**
**90 Long Acre**
**London WC2E 9RA (GB)**

(54) **ACTIVE-ENERGY-RAY-CURABLE COMPOSITION, METHOD FOR MANUFACTURING THREE-DIMENSIONAL SHAPED OBJECT, AND DEVICE FOR MANUFACTURING THREE-DIMENSIONAL SHAPED OBJECT**

(57) Provided is an active-energy-ray-curable composition including: a monomer (A) having a hydrogen-bonding capacity; and a compound (B) having a hydrogen-bonding capacity and containing straight-chain alkyl containing 4 or more carbon atoms, wherein a cured product of the active-energy-ray-curable composition is a solid that exhibits a compressive stress of 2 kPa or higher in response to compression by 1%, and wherein the active-energy-ray-curable composition is a liquid in an environment of 60 degrees C.

## Description

Technical Field

The present invention relates to an active-energy-ray-curable composition, a three-dimensional object producing method, and a three-dimensional object producing apparatus.

Background Art

Techniques called AM (Additive Manufacturing) have been known as techniques for producing three-dimensional stereoscopic objects.

For example, in material jetting methods for producing three-dimensional stereoscopic objects by discharging a liquid-state photocurable resin and laminating layers of the photocurable resin, it is common to employ a method of simultaneously producing a support portion for shape support by laminated object manufacturing, together with producing a model portion by laminated object manufacturing. As the method for removing the support after laminated object manufacturing, there have been proposed a method of producing a support portion using the same material as a model portion and removing the support portion by post-processing such as cutting and polishing (for example, see Patent document 1), and a method of producing a support portion using a water-soluble curable material and removing the support portion by dissolving the support portion in water (for example, see Patent document 2). There is also a method of using a wax as a support material, applying heat to the wax to enable the wax to be discharged by an inkjet method and used for producing a support, and applying heat to the support to liquefy and remove the wax (for example, see Patent document 3). The support portion formed of the wax is liquefied by application of heat. This is an advantageous point because it is possible to remove the support portion easily from a minute portion without damaging the model portion.

Citation List

Patent Document

Patent document 1: Japanese Translation of PCT International Application No. JP-T-2003-535712
Patent document 2: Japanese Unexamined Patent Application Publication No. 2012-111226
Patent document 3: U.S. Patent No. 8575258

Summary of Invention

Technical Problem

The present invention has an object to provide an active-energy-ray-curable composition, a cured product of which has a sufficient shape supporting ability and an excellent removability during production of a three-dimensional object by a stereolithography technique. The present invention further has an object to provide an active-energy-ray-curable composition, a cured product of which can be easily removed with heat.

Solution to Problem

As a result of earnest studies, the present inventors have found that the problems described above can be overcome with an active energy-ray-curable composition described below.

An active-energy-ray-curable composition includes a monomer (A) having a hydrogen-bonding capacity and a compound (B) having a hydrogen-bonding capacity and containing straight-chain alkyl containing 4 or more carbon atoms. A cured product of the active-energy-ray-curable composition is a solid that exhibits a compressive stress of 2 kPa or higher in response to compression by 1%. The active-energy-ray-curable composition is a liquid in an environment of 60 degrees C.

Effects of the Invention

The present invention can provide an active-energy-ray-curable composition, a cured product of which has a sufficient shape supporting ability and an excellent removability. The present invention can further provide an active-energy-ray-curable composition, a cured product of which can be easily removed with heat.

Brief Description of the Drawings

FIG. 1 is a schematic diagram of liquid films produced with a three-dimensional object producing apparatus used in a three-dimensional object producing method of the present invention;
FIG. 2 is a diagram of a three-dimensional object produced by laminating layers of the liquid films illustrated in FIG. 1;
FIG. 3 is an exemplary diagram illustrating an example in which a cured product of an active-energy-ray-curable composition of the present invention is used as a support portion; and
FIG. 4 is an exemplary diagram illustrating another example in which a cured product of an active-energy-ray-curable composition of the present invention is used as a support portion.

Mode for Carrying out the Invention

An embodiment for working the present invention will be described below. So-called persons skilled in the art would easily reach another embodiment by changing or modifying the present invention defined in the claims. Such changes or modifications are intended to be included within the scope of the claims. The following description is intended to illustrate an example embodiment of the present invention, and is not to limit the scope of the claims.

[Active-energy-ray-curable composition]

An active-energy-ray-curable composition of the present invention is suitable as a shape support material (hereinafter may also be referred to as support material) in a laminated object manufacturing method. The active-energy-ray-curable composition contains a monomer (A) having a hydrogen-bonding capacity and a compound (B) having a hydrogen-bonding capacity and containing straight-chain alkyl containing 4 or more carbon atoms. A cured product of the active-energy-ray-curable composition is a solid that exhibits a compressive stress of 2 kPa or higher in response to compression by 1%. The active-energy-ray-curable composition is a liquid in an environment of 60 degrees C. The active-energy-ray-curable composition further contains other components as needed.
The active-energy-ray-curable composition of the present invention is based on the following finding. Existing support portions that are water-soluble have been insufficient in the supporting ability. In the case of producing an object having a large volume with a large-sized object producing apparatus, the insufficiency of the supporting ability is a significant problem. Moreover, when a support portion is dissolved in water, there may be a case where the water in which the support portion is dissolved needs to be treated as an industrial waste.
The active-energy-ray-curable composition of the present invention is also based on the following finding. When existing waxes are used as support portions, there are problems related with the object production accuracy, such as warpage due to shrinkage of the waxes. Furthermore, there is a problem that an apparatus main body such as an ink flow path needs to be heated in addition to an inkjet head, in order to liquefy and discharge the waxes.
The active-energy-ray-curable composition of the present invention is also based on the following finding. When an existing main object is formed using an aqueous curable material, it is preferable that the support portion be water-insoluble, and a water-soluble cured product is insufficient in the supporting ability.

<Monomer (A) having hydrogen-bonding capacity>

Examples of the monomer (A) having a hydrogen-bonding capacity include a monofunctional monomer having a hydrogen-bonding capacity and a multifunctional monomer having a hydrogen-bonding capacity.
Examples of the monomer having a hydrogen-bonding capacity include monomers containing, for example, an amide group, an amino group, a hydroxyl group, a tetramethyl ammonium group, a silanol group, an epoxy group, or a sulfo group.
Examples of polymerization reactions of the monomer include radical polymerization, ionic polymerization, coordination polymerization, and ring-opening polymerization. Radical polymerization is preferable in terms of controlling a polymerization reaction. Hence, an ethylenically unsaturated monomer is preferable as the monomer (A) having a hydrogen-bonding capacity. Above all, a water-soluble ethylenically unsaturated monomer having a high hydrogen-bonding capacity is particularly preferable.

<<Water-soluble monofunctional ethylenically unsaturated monomer having hydrogen-bonding capacity>>

Examples of water-soluble monofunctional monomers having a hydrogen-bonding capacity include: monofunctional vinyl amide group-containing monomers [e.g., N-vinyl-ε-caprolactam, N-vinyl formamide, and N-vinyl pyrrolidone];

monofunctional hydroxyl group-containing (meth)acrylates [e.g., hydroxyethyl (meth)acrylate, hydroxypropyl (meth)acrylate, and 4-hydroxybutyl (meth)acrylate]; hydroxyl group-containing (meth)acrylates [e.g., polyethylene glycol mono(meth)acrylate, monoalkoxy (C1 to C4) polyethylene glycol mono(meth)acrylate, polypropylene glycol mono(meth)acrylate, monoalkoxy (C1 to C4) polypropylene glycol mono(meth)acrylate, and mono(meth)acrylate of PEG-PPG block polymer]; (meth)acrylamide derivatives [e.g., (meth)acrylamide, N-methyl(meth)acrylamide, N-ethyl (meth)acrylamide, N-propyl (meth)acrylamide, N-butyl (meth)acrylamide, N,N'-dimethyl (meth)acrylamide, N-hydroxyethyl (meth)acrylamide, N-hydroxypropyl (meth)acrylamide, and N-hydroxybutyl (meth)acrylamide]; and (meth)acryloylmorpholine. One of these water-soluble monofunctional monomers may be used alone or two or more of these water-soluble monofunctional monomers may be used in combination. Among these water-soluble monofunctional monomers, acrylates and acrylamide derivatives are preferable, and hydroxyethyl acrylate, hydroxypropyl acrylate, and 4-hydroxybutyl acrylate, and acrylamide, acryloylmorpholine, N-methyl acrylamide, N-ethyl acrylamide, N-propyl acrylamide, N-butyl acrylamide, N,N'-dimethyl acrylamide, N,N'-diethyl acrylamide, N-hydroxyethyl acrylamide, N-hydroxypropyl acrylamide, and N-hydroxybutyl acrylamide are more preferable in terms of photoreactivity, and acryloylmorpholine and N-hydroxyethyl acrylamide are preferable in terms of a low skin stimulativeness to a human body.

<<Multifunctional ethylenically unsaturated monomer having hydrogen-bonding capacity>>

Examples of multifunctional ethylenically unsaturated monomers having a hydrogen-bonding capacity include bifunctional group monomers and trifunctional or higher monomers.

Examples of the bifunctional group monomers include tripropylene glycol di(meth)acrylate, triethylene glycol di(meth)acrylate, tetraethylene glycol di(meth)acrylate, polypropylene glycol di(meth)acrylate, neopentyl glycol hydroxypivalic acid ester di(meth)acrylate, hydroxypivalic acid neopentyl glycol ester di(meth)acrylate, 1.3-butanediol di(meth)acrylate, 1.4-butanediol di(meth)acrylate, 1.6-hexanediol di(meth)acrylate, 1,9-nonanediol di(meth)acrylate, diethylene glycol di(meth)acrylate, neopentyl glycol di(meth)acrylate, tripropylene glycol di(meth)acrylate (TPGDA), caprolactone-modified hydroxypivalic acid neopentyl glycol ester di(meth)acrylate, propoxylated neopentyl glycol di(meth)acrylate, polyethylene glycol 200 di(meth)acrylate, and polyethylene glycol 400 di(meth)acrylate.

Examples of the trifunctional or higher monomers include triallyl isocyanate and tris(2-hydroxyethyl)isocyanurate tri(meth)acrylate.

One of these multifunctional ethylenically unsaturated monomers may be used alone or two or more of these multifunctional ethylenically unsaturated monomers may be used in combination.

<Compound (B) having hydrogen-bonding capacity and containing straight-chain alkyl containing 4 or more carbon atoms>

Examples of the compound (B) having a hydrogen-bonding capacity and containing straight-chain alkyl containing 4 or more carbon atoms include: higher alcohols [e.g., 1-hexanol, 1-decanol, and 1-dodecanol]; surfactants containing a hydroxyl group and straight-chain alkyl containing 4 or more carbon chains [e.g., glycerol monostearate, glycerol distearate, glycerol tristearate, sorbitan monolaurate, sorbitan monopalmitate, sorbitan dilaurate, sorbitan trioleate, and polyoxyethylene sorbitan monolaurate]; alcohols containing 2 or more hydroxyl groups and straight-chain alkyl containing 4 or more carbon chains [e.g., diol: 1,5-pentanediol and 1,6-hexanediol, triol: 1,2,5-pentanetriol]; amines containing straight-chain alkyl containing 4 or more carbon chains [e.g., 1-hexylamine, 1-octylamine, and 1-decaneamine]; and sulfonic acids containing straight-chain alkyl containing 4 or more carbon chains [e.g., hexyl p-toluenesulfonate, octyl p-toluenesulfonate, and p-dodecylbenzene sulfonic acid]. One of these compounds may be used alone or two or more of these compounds may be used in combination.

Higher alcohols are advantageous in that the higher alcohols have a hydrogen bond because of the presence of a hydroxyl group, and can easily crystallize because of a simple structure. Diols are advantageous in that the diols have an even stronger hydrogen-bonding capacity because of the presence of 2 hydroxyl groups, and a cured product of the diols is strong.

It is preferable that the active-energy-ray-curable composition of the present invention further contain a photopolymerization initiator (C).

<Photopolymerization initiator (C)>

As the photopolymerization initiator (C), an arbitrary substance that produces radicals in response to irradiation of light (particularly, an ultraviolet ray having a wavelength of from 220 nm through 400 nm) can be used.

Examples of such substances include acetophenone, 2, 2-diethoxyacetophenone, p-dimethylaminoacetophenone, benzophenone, 2-chlorobenzophenone, p,p'-dichlorobenzophenone, p,p-bisdiethylaminobenzophenone, Michler's ketone, benzyl, benzoin, benzoin methyl ether, benzoin ethyl ether, benzoin isopropyl ether, benzoin-n-propyl ether, benzoin isobutyl ether, benzoin-n-butyl ether, benzyl methyl ketal, thioxanthone, 2-chlorothioxanthone, 2-hydroxy-2-methyl-1-phenyl-1-one, 1-(4-isopropylphenyl)2-hydroxy-2-methyl propan-1-one, methyl benzoyl formate, 1-hydroxycyclohexyl phenyl ketone, azobis isobutyronitrile, benzoyl peroxide, and di-tert-butyl peroxide. One of these substances may be used alone or two or more of these substances may be used in combination. It is preferable to select a photopolymerization initiator matching the ultraviolet wavelength of an ultraviolet ray irradiator.

It is preferable that the active-energy-ray-curable composition of the present invention contain the compound (B) having a hydrogen-bonding capacity and containing straight-chain alkyl containing 4 or more carbon atoms in an amount of from 20% by mass through 80% by mass and more preferably from 40% by mass through 60% by mass relative to the total amount of the active-energy-ray-curable composition.

It is preferable that the active-energy-ray-curable composition of the present invention contain the photopolymerization initiator (C) in an amount of from 0.1% by mass through 10% by mass and more preferably from 0.5% by mass through 6% by mass relative to the total amount of the active-energy-ray-curable composition.

In order to obtain a cured product from the active-energy-ray-curable composition, it is preferable to cure the active-energy-ray-curable composition by irradiation with an ultraviolet ray in an exposure amount of 200 mJ/cm$^2$ or higher using an ultraviolet ray irradiator.

<Active energy ray>

Active energy rays used for curing an active-energy-ray-curable composition of the present invention are not particularly limited, so long as they are able to give necessary energy for allowing polymerization reaction of polymerizable components in the composition to proceed. Examples of the active energy rays include electron beams, $\alpha$-rays, $\beta$-rays, $\gamma$-rays, and X-rays, in addition to ultraviolet rays. When a light source having a particularly high energy is used, polymerization reaction can be allowed to proceed without a polymerization initiator. In addition, in the case of irradiation with ultraviolet ray, mercury-free is preferred in terms of protection of environment. Therefore, replacement with GaN-based semiconductor ultraviolet light-emitting devices is preferred from industrial and environmental point of view. Furthermore, ultraviolet light-emitting diode (UV-LED) and ultraviolet laser diode (UV-LD) are preferable as an ultraviolet light source. Small sizes, long time working life, high efficiency, and high cost performance make such irradiation sources desirable.

It is preferable that the monomer (A) having a hydrogen-bonding capacity and the compound (B) having a hydrogen-bonding capacity and containing straight-chain alkyl containing 4 or more carbon atoms satisfy the following condition.

<Condition>

When a liquid mixture of the monomer (A) (50 parts by mass), the compound (B) (50 parts by mass), and the photopolymerization initiator (C) (5 parts by mass) is irradiated with 500 mJ/cm$^2$ of ultraviolet ray with an ultraviolet ray irradiator, the obtained cured product is a solid that exhibits a compressive stress of 2 kPa or higher in response to compression by 1% in an environment of 25 degrees C.

When the monomer (A) containing the photopolymerization initiator (C) is irradiated with an ultraviolet ray and becomes a polymer, the compound (B) binds with the polymer via a hydrogen bond. The bound compound (B) becomes a solid by carbon chains being arranged in an environment of 25 degrees C.

The monomer (A) and the compound (B) have a hydrogen-bonding capacity. There are a very large number of combinations of the monomer (A) having a hydrogen-bonding capacity and the compound (B) having a hydrogen-bonding capacity. However, the polymer and the compound (B) are unable to sufficiently bind with each other with a weak hydrogen-bonding capacity, and may be unable to become a solid through ultraviolet ray irradiation. Hence, by testing the condition described above, it is possible to easily explore a combination of the monomer (A) and the compound (B) that are bound with each other via a hydrogen bond.

The condition described above is used for exploring combinations of the monomer (A) and the compound (B). All active-energy-ray-curable compositions that use the monomer (A) and the compound (B) that satisfy the condition described above do not always qualify as the active-energy-ray-curable composition of the present invention. The physical properties of the active-energy-ray-curable composition of the present invention are also related with the blending amounts of the monomer (A) and the compound (B).

It is preferable that the compound (B) having a hydrogen-bonding capacity and containing straight-chain alkyl containing 4 or more carbon atoms be compatible with the monomer (A) having a hydrogen-bonding capacity and be a non-reactive compound.

What is meant by that the compound (B) having a hydrogen-bonding capacity and containing straight-chain alkyl containing 4 or more carbon atoms is compatible with the monomer (A) having a hydrogen-bonding capacity is that the compound (B) and the monomer (A) have affinity with each other and become a solution or a mixture.

In the present invention, what is meant by that the compound (B) having a hydrogen-bonding capacity and containing straight-chain alkyl containing 4 or more carbon atoms is a non-reactive compound is that the compound (B) does not undergo a chemical reaction even when irradiated with an ultraviolet ray.

When the compound (B) having a hydrogen-bonding capacity and containing straight-chain alkyl containing 4 or more carbon atoms is compatible with the monomer (A) having a hydrogen-bonding capacity, the functionality sought after, such as curing is more easily obtained, because no interface is to be formed. Moreover, a good dischargeability is obtained.

It is preferable that the compound (B) be non-reactive, because the compound (B) does not undergo a chemical reaction with a photopolymerization initiator and does not inhibit the reaction of the monomer.

The surface tension of the active-energy-ray-curable composition is not particularly limited, may be appropriately selected depending on the intended purpose, and is preferably, for example, from 20 mN/m through 45 mN/m and more preferably from 25 mN/m through 34 mN/m at 25 degrees C.

When the surface tension of the active-energy-ray-curable composition is 20 mN/m or lower, discharging is stable during object production, with no bending of the discharging direction or no empty discharging. When the surface tension of the active-energy-ray-curable composition is 45 mN/m or lower, for example, discharging nozzles for object production can be filled with the liquid completely, when filling the nozzles with the liquid.

The surface tension can be measured with, for example, a surface tensiometer (automatic contact angle gauge DM-701, available from Kyowa Interface Science Co., Ltd.).

- Viscosity-

The viscosity of the active-energy-ray-curable composition is preferably 100 mPa·s or lower at 25 degrees C, more preferably from 3 mPa·s through 20 mPa·s and particularly preferably from 6 mPa·s through 12 mPa·s at 25 degrees C.

When the viscosity of the active-energy-ray-curable composition is higher than 100 mPa·s, the active-energy-ray-curable composition may not be discharged even when a head is heated.

The viscosity can be measured with, for example, a rotational viscometer (VISCOMATE VM-150III, available from Toki Sangyo Co., Ltd.) in an environment of 25 degrees C.

-Viscosity change rate-

The viscosity change rate of the active-energy-ray-curable composition between before and after the active-energy-ray-curable composition is left to stand at 50 degrees C for 2 weeks is preferably 20% or lower and more preferably 10% or lower.

When the viscosity change rate of the active-energy-ray-curable composition is 10% or lower, the active-energy-ray-curable composition has an appropriate storage stability and a good discharging stability.

The viscosity change rate between before and after being left to stand at 50 degrees C for 2 weeks can be measured in the manner described below.

The active-energy-ray-curable composition poured in a widemouthed bottle (50 mL) formed of polypropylene is left to stand in a thermostat bath of 50 degrees C for 2 weeks, taken out from the thermostat bath, and left to stand until the active-energy-ray-curable composition becomes room temperature (25 degrees C). Then, the viscosity of the active-energy-ray-curable composition is measured. The viscosity change rate is calculated according to the formula described below, where viscosity before storage refers to the viscosity of the active-energy-ray-curable composition before put in the thermostat bath and viscosity after storage refers to the viscosity of the active-energy-ray-curable composition after taken out from the thermostat bath. The viscosity before storage and the viscosity after storage can be measured with an R-type viscometer (available from Toki Sangyo Co., Ltd.) at 25 degrees C.

$$\text{Viscosity change rate } (\%) = [(\text{viscosity after storage}) - (\text{viscosity before storage})]/(\text{viscosity before storage}) \times 100$$

-Other components-

The other components that may be contained in the active-energy-ray-curable composition are not particularly limited and may be appropriately selected depending on the intended purpose. Examples of the other components include a polymerization inhibitor, a mineral dispersible in the active-energy-ray-curable composition, a polymerizable monomer, a thermal polymerization initiator, a colorant, an antioxidant, a chain-transfer agent, an age resistor, a cross-linking promoter, an ultraviolet absorber, a plasticizer, an antiseptic, and a dispersant.

--Polymerization inhibitor--

Examples of the polymerization inhibitor include: phenol compounds [e.g., hydroquinone, hydroquinone monomethyl ether, 2,6-di-t-butyl-p-cresol, 2,2-methylene-bis-(4-methyl-6-t-butylphenol), and 1,1,3-tris-(2-methyl-4-hydroxy-5-t-butylphenyl)butane]; sulfur compounds [e.g., dilaurylthio dipropionate]; phosphorus compounds [e.g., triphenyl phosphite]; and amine compounds [e.g., phenothiazine].

The amount of the polymerization inhibitor to be used based on the total mass of the active-energy-ray-curable composition is typically 5% or lower, and preferably from 0.01% through 3% in terms of the stability of the monomer and the polymerization speed.

--Mineral dispersible in active-energy-ray-curable composition--

The mineral dispersible in the active-energy-ray-curable composition is not particularly limited. Examples of the mineral include a layered clay mineral.

Examples of the layered clay mineral include: smectite such as montmorillonite, beidellite, hectorite, saponite, nontronite, and stevensite; vermiculite; bentonite; and layered sodium silicate such as kanemite, kenyanite, and macanite. The layered clay mineral may be a natural mineral or may be produced by a chemical synthesizing method.

The layered clay mineral may be surface-treated with an organic substance. When a layered inorganic substance such as a layered clay mineral is treated with an organic cationic compound, interlayer cations may be ion-exchanged with cationic groups such as quaternary salts. Examples of the cations of the layered clay mineral include metal cations such as sodium cations and calcium cations. The layered clay mineral treated with an organic cationic compound is more swellable and dispersible in the polymer and the polymerizable monomer. Examples of the layered clay mineral treated with an organic cationic compound include LUCENTITE SERIES (available from Co-op Chemical Co., Ltd.). More specific examples of LUCENTITE SERIES (available from Co-op Chemical Co., Ltd.) include LUCENTITE SPN, LUCENTITE SAN, LUCENTITE SEN, and LUCENTITE STN.

--Polymerizable monomer--

The polymerizable monomer is not particularly limited, and examples of the polymerizable monomer include (meth)acrylates. Examples of the (meth)acrylates include 2-ethylhexyl (meth)acrylate (EHA), isobornyl (meth)acrylate, 3-methoxybutyl (meth)acrylate, lauryl (meth)acrylate, 2-phenoxyethyl (meth)acrylate, isodecyl (meth)acrylate, isooctyl (meth)acrylate, tridecyl (meth)acrylate, caprolactone (meth)acrylate, and ethoxylated nonylphenol (meth)acrylate. One of these polymerizable monomers may be used alone or two or more of these polymerizable monomers may be used in combination.

--Thermal polymerization initiator--

The thermal polymerization initiator is not particularly limited and may be appropriately selected depending on the intended purpose. Examples of the thermal polymerization initiator include azo-based initiators, peroxide initiators, persulfate initiators, and redox (oxidoreduction) initiators. A photopolymerization initiator is preferred to a thermal polymerization initiator in terms of storage stability.

Examples of the azo-based initiators include VA-044, VA-46B, V-50, VA-057, VA-061, VA-067, VA-086, 2,2'-azobis(4-methoxy-2,4-dimethylvaleronitrile) (VAZO 33), 2,2'-azobis(2-amidinopropane)dihydrochloride (VAZO 50), 2,2'-azobis(2,4-dimethylvaleronitrile) (VAZO 52), 2,2'-azobis(isobutyronitrile) (VAZO64), 2,2'-azobis-2-methylbutyronitrile (VAZO 67), and 1,1-azobis(1-cyclohexanecarbonitrile) (VAZO 88) (all available from DuPont Chemicals Company), and 2,2'-azobis(2-cyclopropylpropionitrile) and 2,2'-azobis(methylisobutyrate) (V-601) (available from Wako Pure Chemical Industries, Ltd.).

Examples of the peroxide initiators include benzoyl peroxide, acetyl peroxide, lauroyl peroxide, decanoyl peroxide, dicetyl peroxydicarbonate, di(4-t-butylcyclohexyl)peroxydicarbonate (PERKADOX 16S) (available from Akzo Nobel), di(2-ethylhexl)peroxydicarbonate, t-butyl peroxypivalate (LUPERSOL 11) (available from Elf Atochem), t-butyl peroxy-2-ethyl hexanoate (TRIGONOX 21-C50) (available from Akzo Nobel), and dicumyl peroxide.

Examples of the persulfate initiators include potassium persulfate, sodium persulfate, and ammonium persulfate.

Examples of the redox (oxidoreduction) initiators include a combination of the persulfate initiator and a reducing agent such as sodium hydrogen metasulfite and sodium hydrogen sulfite, a system based on the organic peroxide and tertiary amine (e.g., a system based on benzoyl peroxide and dimethyl aniline), and a system based on organic hydroperoxide and a transition metal (e.g., a system based on cumene hydroperoxide and cobalt naphthenate).

--Colorant--

Examples of the colorant include pigments and dyes. Pigments include organic and inorganic pigments. Examples of the pigments are presented below.

Examples of the organic pigments include azo pigments, polycyclic pigments, azine pigments, daylight fluorescent pigments, nitroso pigments, nitro pigments, and natural pigments.

Examples of the inorganic pigments include metal oxides (e.g., iron oxide, chromium oxide, and titanium oxide), and carbon black.

-- Antioxidant--

Examples of the antioxidant include phenol compounds {e.g., monocyclic phenol (e.g., 2,6-di-t-butyl-p-cresol), bisphenol [e.g., 2,2'-methylene bis(4-methyl-6-t-butylphenol)], and polycyclic phenol [e.g., 1,3,5-trimethyl-2,4,6-tris(3,5-di-t-butyl-4-hydroxybenzyl)benzene]}, sulfur compounds (e.g., dilauryl 3,3'-thiodipropionate), phosphorus compounds (e.g., triphenyl phosphite), and amine compounds (e.g., octylated diphenylamine).

--Chain-transfer agent--

Examples of the chain-transfer agent include hydrocarbons [e.g., C6 to C24 compounds such as aromatic hydrocarbons (e.g., toluene and xylene) and unsaturated aliphatic hydrocarbons (e.g., 1-butene and 1-nonene)]; halogenated hydrocarbons (e.g., C1 to C24 compounds such as dichloromethane and carbon tetrachloride); alcohols (e.g., C1 to C24 compounds such as methanol and 1-butanol); thiols (e.g., C1 to C24 compounds such as ethyl thiol and 1-octyl thiol); ketones (e.g., C3 to C24 compounds such as acetone and methyl ethyl ketone); aldehydes (e.g., C2 to C18 compounds such as 2-methyl-2-propyl aldehyde and 1-pentyl aldehyde); phenols (e.g., C6 to C36 compounds such as phenols and m-, p- and o-cresols); quinones (e.g., C6 to C24 compounds such as hydroquinone); amines (e.g., C3 to C24 compounds such as diethyl methyl amine and diphenyl amine); and disulfides (e.g., C2 to C24 compounds such as diethyl disulfide and di-1-octyl disulfide).

The active-energy-ray-curable composition (shape supporting material) is a shape supporting liquid material before solidified. However, the active-energy-ray-curable composition of the present invention may also be the shape supporting liquid material after solidified. That is, the active-energy-ray-curable composition of the present invention encompasses an active-energy-ray-curable composition (shape supporting solid material) that contains a polymer (A) having a hydrogen-bonding capacity and the compound (B) having a hydrogen-bonding capacity and containing straight-chain alkyl containing 4 or more carbon atoms, is a solid that exhibits a compressive stress of 2 kPa or higher in response to compression by 1% in an environment of 25 degrees C, and is a liquid in an environment of 60 degrees C. The polymer (A) having a hydrogen-bonding capacity is a polymer of the monomer (A) having a hydrogen-bonding capacity.

The active-energy-ray-curable composition is preferably a liquid having a viscosity of 100 mPa·s or lower in an environment of 60 degrees C.

The active-energy-ray-curable composition of the present invention is applied in the form of a liquid during object production, and solidified in order to support an object. For removal, the active-energy-ray-curable composition is heated. The shape supporting solid material is supplied in the form of an active-energy-ray-curable composition (shape supporting material) that is solid, and applied (by IJ discharging) under heating during object production, and solidified again in order to support an object. After object production, the shape supporting solid material can be heated again to be liquefied and removed.

<Supporting force of active-energy-ray-curable composition (shape supporting material)>

The supporting force of a cured product of the shape supporting liquid material (hereinafter, the cured product may also be referred to as "shape supporting solid material", "support portion", or "cured product of a support material") is a property of the shape supporting solid material of supporting a model portion and can be expressed by a compressive stress in response to compression by 1%. As the supporting force of the shape supporting solid material, the shape supporting solid material needs to be a solid that exhibits a compressive stress of 2 kPa or higher, preferably from 2 kPa through 1,000 kPa, and more preferably from 2 kPa through 300 kPa in response to compression by 1% in an environment of 25 degrees C in terms of the object production accuracy of an object produced by stereolithography and the solubility of a support portion. It is possible to adjust the supporting force of the shape supporting solid material to the range described above, by selecting the kinds and the amounts of use of the polymer (A) and the compound (B) that constitute the shape supporting solid material. A compressive stress at 1% strain can be measured with a universal tester (available from Shimadzu Corporation, AG-I).

The supporting force of the shape supporting solid material of the present invention is high because of the orientation of straight-chain alkyl. It can be inferred that a high supporting force is secured by bonding via a hydrogen bond between the polymer obtained through polymerization of the monomer (A) having a hydrogen-bonding capacity and the compound (B) having a hydrogen-bonding capacity and containing straight-chain alkyl containing 4 or more carbon atoms, and by the orientation of straight-chain alkyl.

<Removability of shape supporting solid material>

As described above, the supporting force of the shape supporting solid material of the present invention is attributable to the orientation of straight-chain alkyl. Hence, temperature application, which causes the orientation of straight-chain alkyl to collapse, enables the shape supporting solid material to be liquefied and removed. By the shape supporting solid material changing to a liquid in an environment of 60 degrees C, the support portion can be easily removed.
For example, the shape supporting solid material can be removed by immersion in hot water of about 60 degrees C. When the support portion has slightly remained on a model portion, it is preferable to apply, for example, ultrasonic waves during immersion.

<Embodiment>

A three-dimensional object producing method of the present invention uses the active-energy-ray-curable composition of the present invention.
The three-dimensional object producing method of the present invention is a method of forming a model portion and a support portion and subsequently removing the support portion, to produce a three-dimensional object. The method includes forming a support portion using the active-energy-ray-curable composition of the present invention, and subsequently removing the support portion.
For the removing, it is preferable to perform the removing by heating the support portion to liquefy the support portion.
The method includes a step of forming a liquid film formed of a liquid containing the active-energy-ray-curable composition of the present invention and a step of curing the liquid film. It is preferable to perform the step of forming a liquid film by an inkjet method or with a dispenser.
A three-dimensional object producing apparatus of the present invention includes a liquid film forming unit configured to form a liquid film using the active-energy-ray-curable composition of the present invention while controlling the coating position and the coating amount of the active-energy-ray-curable composition, and a unit configured to cure the liquid film.
It is preferable that the liquid film forming unit be of an inkjet type.
The active-energy-ray-curable composition of the present invention can be used as an active-energy-ray-curable composition for obtaining a cured product that can support a three-dimensional object during production of the object (hereinafter may also be referred to as "model portion" or "cured product of a model material") using an aqueous curable material in the three-dimensional object producing method. Furthermore, with change of the polarities of the monomer (A) having a hydrogen-bonding capacity and the compound (B) having a hydrogen-bonding capacity and containing straight-chain alkyl containing 4 or more carbon atoms, the active-energy-ray-curable composition can also be used as an active-energy-ray-curable composition for obtaining a cured product that can support a three-dimensional object during production of the object using an oil-based curable material.
A specific embodiment for producing a three-dimensional object using the active-energy-ray-curable composition of the present invention will be described below.
First, surface data or solid data representing a three-dimensional shape designed by three-dimensional CAD or a three-dimensional shape scanned with a three-dimensional scanner or a digitizer is converted into a STL format and input to a laminated object manufacturing apparatus.
Next, based on the input data, an object producing orientation for producing a three-dimensional shape to be produced is determined. The object producing orientation is not particularly limited, and, typically, an orientation that has the smallest size in the Z-direction (height direction) is selected.
After the object producing orientation is determined, the projected areas of the three-dimensional shape on an X-Y plane, an X-Z plane, and a Y-Z plane are obtained. The obtained block shape is sliced in the Z-direction at intervals determined by the thickness of one layer. The thickness of one layer is different depending on the materials used, but is typically about from 20 micrometers through 60 micrometers. When there is one object to be produced, this block shape is disposed in the center of a Z stage (which is an object placing table that is configured to lift down by a distance corresponding to one layer every time one layer is formed). In the case of producing a plurality of objects simultaneously, block shapes are disposed on the Z stage, or alternatively, the block shapes may be stacked one upon another. These operations of block shaping, slice data (contour data) generation, and Z stage positioning may be automated with designation of the materials to be used.

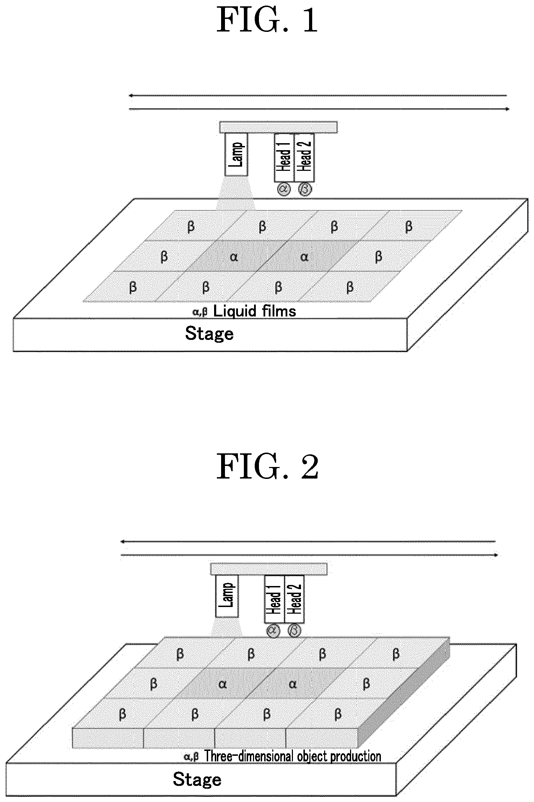

Next, an object producing step is performed. Different heads 1 and 2 (FIG. 1) are moved bidirectionally, to discharge a model material precursor liquid α and the active-energy-ray-curable composition β and form dots. By forming continuous dots, it is possible to form liquid films at desired positions. With irradiation of the liquid films with an ultraviolet (UV) ray of light to cure the liquid films, a film of the model material and a film of the support material can be formed at desired positions.

After a film of the model material and a film of the support material are formed for one layer, a stage (FIG. 1) lifts down by a distance corresponding to the thickness of one layer. Again, continuous dots are formed over the film of the model material and the film of the support material, to form liquid films at desired positions. With irradiation of the liquid films with an ultraviolet (UV) ray of light to cure the liquid films, a film of the model material and a film of the support material are formed at desired positions. Through repetition of this layer lamination sequence, a three-dimensional object can be produced as illustrated in FIG. 2.

The support portion can be removed with heat from the object produced in this three-dimensional object producing manner, and a desired model portion can be obtained.

Examples

The present invention will be described by way of Examples. The present invention should not be construed as being limited to these Examples.

<Examples 1 to 5, and Comparative Examples 1 to 3>

Materials were uniformly mixed according to the formulation (part by mass) presented in Table 1 below, to produce active-energy-ray-curable compositions to be used in Examples 1 to 5 and Comparative Examples 1 to 3.

When the active-energy-ray-curable composition of each of Examples 1 to 5 and Comparative Examples 1 and 2 was irradiated with 500 mJ/cm$^2$ of ultraviolet ray by an ultraviolet ray irradiator, it was confirmed that the obtained cured product was a solid that exhibited a compressive stress of 2 kPa or higher in response to compression by 1% in an environment of 25 degrees C.

As for Comparative Example 3, when a liquid mixture of the monomer (A) (50 parts by mass), the compound (B) (50 parts by mass), and the photopolymerization initiator (C) (5 parts by mass) presented in Table 1 below was irradiated with 500 mJ/cm$^2$ of ultraviolet ray by an ultraviolet ray irradiator, the obtained cured product exhibited a compressive stress of lower than 2 kPa in response to compression by 1% in an environment of 25 degrees C.

The signs in Table 1 below stand for the followings.

| | |
|---|---|
| A-1: | Acryloylmorpholine (available from KJ Chemicals Corp.) |
| A-2: | Hydroxyethyl acrylamide (available from KJ Chemicals Corp.) |
| A-3: | Diethyl acrylamide (available from KJ Chemicals Corp.) |
| B-1: | 1-Hexanol (available from Tokyo Chemical Industry Co., Ltd.) |
| B-2: | 1-Hexylamine (available from Tokyo Chemical Industry Co., Ltd.) |
| B-3: | 1,5-Pentanediol (available from Tokyo Chemical Industry Co., Ltd.) |
| C: | 1-Hydroxycyclohexyl phenyl ketone [Product name: IRGACURE 184] (available from BASF) |
| D: | Phenothiazine (available from Tokyo Chemical Industry Co., Ltd.) |

The viscosity of the obtained active-energy-ray-curable compositions was measured with a rotational viscometer (VISCOMATE VM-150III, available from Toki Sangyo Co., Ltd.) in an environment of 25 degrees C.

Furthermore, after the obtained cured products changed to liquids at 60 degrees C, the viscosity of the liquids at 60 degrees C was also measured with a rotational viscometer (VISCOMATE VM-150III, available from Toki Sangyo Co., Ltd.).

The obtained cured products (support portions) were evaluated according to <Evaluation items> described below. The results are presented in the Evaluation item section of Table 1 below.

<Evaluation items>

Each active-energy-ray-curable composition presented in Table 1 below was picked up in an amount of 2 g in a silicon frame having a size of 20 mm × 20 mm × 5 mm, and irradiated with 500 mJ/cm$^2$ of ultraviolet ray by an ultraviolet ray irradiator, to obtain a support portion, which was a cured product. The support portion was evaluated in the manners described below.

(1) Change from solid to liquid at 60 degrees C

The support portion (2 g) was put in a thermostat bath of 60 degrees C, to visually judge whether the support portion would change from the solid to a liquid. The score given when the support portion changed from the solid to a liquid is expressed as B, and the score given when the support portion did not change from the solid to a liquid is expressed as D.

(2) Removability of support portion

A silicon rubber having a size of 20 mm × 20 mm × 5 mm was processed into a product that had a through-hole penetrating the center portion in the vertical direction and having a diameter of 5 mm. This product was put on a glass plate in a manner that the hole faced upward, and the active-energy-ray-curable composition (supporting material) was poured into the hole to fill the hole and irradiated with 500 mJ/cm$^2$ of ultraviolet ray by an ultraviolet ray irradiator, to obtain a cured product of the support material (support portion). Next, the cured product of the support material was removed by being put in hot water of 60 degrees C together with the processed silicon rubber, and being subjected to application of ultrasonic waves (available from As One Corporation: ASU-6) for 30 minutes. Thirty minutes later, the silicon rubber was taken out, to evaluate the removability of the support portion according to the criteria described below.

<Evaluation criteria>

B: The removability of the support portion was good (the hole was completely through).
C: The removability of the support portion was insufficient (the hole was not through at some portions).
D: The removability of the support portion was poor (the hole was clogged with the swollen cured product).

(3) Supporting force of support portion

In an environment of 25 degrees C, a universal tester (available from Shimadzu Corporation, AG-I), a load cell for 1 kN, and a compression jig for 1 kN were installed, and the cured product of the support material (support portion) (2 g) produced into a shape of 20 mm × 20 mm × 5 mm was set. A stress relative to a compression applied to the load cell was recorded by a computer, to plot the stress relative to the amount of displacement. Based on the stress in response to compression by 1%, the supporting force was evaluated according to the criteria described below.

<Evaluation criteria>

A: The stress was 10 kPa or higher (the support portion had a sufficient supporting force).
B: The stress was 2 kPa or higher but lower than 10 kPa (The support portion had a supporting force).
D: The stress was lower than 2 kPa (the support portion had an insufficient supporting force).

The evaluation results of the evaluation items (1) to (3) are presented in Table 1 below.

Table 1

| | | | Ex. | | | | | Comp. Ex. | | |
|---|---|---|---|---|---|---|---|---|---|---|
| | | | 1 | 2 | 3 | 4 | 5 | 1 | 2 | 3 |
| Blending amount (part by mass) | Monomer (A) | A-1 | 25 | 48 | 48 | 24 | 48 | 15 | 80 | - |
| | | A-2 | - | - | - | 24 | - | - | - | - |
| | | A-3 | - | - | - | - | - | - | - | 48 |
| | Compound (B) | B-1 | 72 | 48 | - | 48 | - | 82 | 17 | 48 |
| | | B-2 | - | - | 48 | - | - | - | - | - |
| | | B-3 | - | - | - | - | 48 | - | - | - |
| | Photopolymerization initiator | C | 2.9 | 3.9 | 3.9 | 3.9 | 3.9 | 2.9 | 2.9 | 2.9 |
| | Polymerization inhibitor | D | | 0.1 | 0.1 | 0.1 | 0.1 | 0.1 | 0.1 | 0.1 |
| Evaluation items | Liquid viscosity (mPa·s) at 25 degrees C | | 6.4 | 7.3 | 4.8 | 12.1 | 30 | 5.8 | 10.6 | 3.7 |
| | Liquid viscosity (mPa·s) at 60 degrees C | | 5.2 | 6.3 | 4.4 | 10.6 | 12.1 | 3.2 | - | - |
| | (1) Change from solid to liquid at 60 degrees C | | B | B | B | B | B | B | D | D |
| | (2) Removability | | B | B | B | B | B | B | C | B |
| | (3) Supporting force of support portion | | B | A | A | A | A | D | A | D |

<Evaluation results>

The evaluation results are presented in the Evaluation items section in Table 1 above. Here, in Examples 1 to 5, the solid changed to a liquid in an environment of 60 degrees C, and the support portion was able to be easily removed. In all of Examples 1 to 5, the removability was good. Furthermore, in Examples 2 to 5, the support portion had a sufficient supporting force, and was able to exert a sufficient supporting force even during production of a large-volume model portion that has been difficult to produce because existing support portions have been insufficient in the supporting force.
In Comparative Example 1, the amount of 1-hexanol was large, and acryloylmorpholine, which was the polymer, was not able to sufficiently carry 1-hexanol, resulting in an insufficient supporting force of the support portion. In Comparative Example 2, the amount of acryloylmorpholine, which was the polymer, was large, and a liquid having a desired viscosity was not obtained in the environment of 60 degrees C. In Comparative Example 3, steric hindrance occurred due to the presence of two ethyl groups on nitrogen of diethyl acrylamide, resulting in hindrance of hydrogen bonding with a hydroxyl group of 1-hexanol. This was considered the reason for insufficient curing.

<Example 6>

Next, a three-dimensional object was produced by an inkjet method using the active-energy-ray-curable composition produced in Example 2.
A hydrogel precursor described in Japanese Unexamined Patent Application Publication No. 2015-136895 was used as a model material.

--Preparation of hydrogel precursor--

As an initiator liquid, a solution was prepared by dissolving a photopolymerization initiator (IRGACURE 184, available from BASF) (2 parts by mass) in methanol (98 parts by mass).
Next, to pure water (195 parts by mass) under stirring, synthetic hectorite (LAPONITE XLG, available from Rock Wood) having a composition $[Mg_{5.34}Li_{0.66}Si_8O_{20}(OH)_4]Na_{0.66}$ (8 parts by mass) was added little by little as a water-swellable layered clay mineral, followed by stirring, to produce a dispersion liquid. To the obtained dispersion liquid, N,N-dimethylacrylamide (available from Wako Pure Chemical Industries., Ltd.) (20 parts by mass) having been passed through an activated alumina column for removal of a polymerization inhibitor was added as a polymerizable monomer. Further, sodium dodecyl sulfate (available from Wako Pure Chemical Industries, Ltd.) (0.2 parts by mass) was added as a surfactant and mixed.

Next, to the resultant under cooling in an ice bath, the initiator liquid (0.5 parts by mass) was added, followed by stirring and mixing, and then vacuum degassing for 10 minutes. Subsequently, the resultant was subjected to filtration to remove, for example, impurities, to obtain a homogeneous hydrogel precursor.

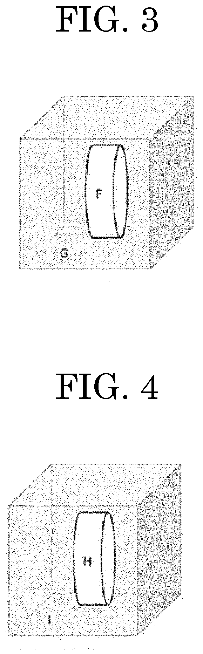

The hydrogel precursor and the active-energy-ray-curable composition produced in Example 2 were used in the head 1 and the head 2 illustrated in FIG. 1 respectively, and a three-dimensional object was produced as illustrated in FIG. 3 in the same manner as in the embodiment described above. In FIG. 3, a sign F denotes a model portion, and a sign G denotes a support portion. As a result, the support portion having a cubic shape supported the hydrogel, which was the model portion having a circular-columnar shape, and formed an interface with the model portion. Detachability and an object production accuracy at the interface were good. The obtained object was left to stand still in a thermostat bath of 60 degrees C for 10 minutes and heated. As a result, the support portion was liquefied, and it was possible to easily obtain the model portion. The support portion that had slightly remained on the model portion was removed by application of ultrasonic waves (available form As One Corporation: ASU-6) for 5 minutes in hot water of 60 degrees C. The obtained model portion was not rough on the surface that had contacted the support portion, and it was possible to remove the support portion without residue.

<Example 7>

Next, a three-dimensional object was produced by an inkjet method using the active-energy-ray-curable composition produced in Example 5.

A model material precursor described in Japanese Unexamined Patent Application Publication No. 2012-111226 was used as a model material.

--Preparation of model material precursor--

2-Hydroxyethyl acrylate-caprolactone adduct (product name: "PLACCEL FA-4D", available from Daicel Corporation, the number of moles added: 4) (100 parts by mass), a nurated product of IPDI (product name "VESTANAT T1890", available from Degussa Japan Co., Ltd.) (64 parts by mass), and as a urethanation catalyst, bismuth tri(2-ethylhexanoate) (50% 2-ethylhexanoic acid solution) (0.03 parts by mass) were filled in a reaction vessel and allowed to undergo a reaction at 80 degrees C for 12 hours, to obtain urethane acrylate.

The urethane acrylate (20 parts by mass), isobornyl acrylate (available from Kyoeisha Chemical Co., Ltd.) (70 parts by mass), dicyclopentane dimethylol diacrylate (available from Kyoeisha Chemical Co., Ltd.) (10 parts by mass), 1,3,5-trimethylbenzoyl diphenylphosphine oxide (available from BASF) (5 parts by mass), and carbon black (product name "MHI BLACK #220", available from Mikuni Color Ltd.) (0.05 parts by mass) were put in a beaker and uniformly mixed, to obtain a model material precursor.

The model material precursor and the active-energy-ray-curable composition produced in Example 5 were used in the head 1 and the head 2 illustrated in FIG. 1 respectively, and a three-dimensional object was produced as illustrated in FIG. 4 in the same manner as in the embodiment described above. In FIG. 4, a sign H denotes a model portion, and a sign I denotes a support portion. As a result, the support portion having a cubic shape supported the model portion having a circular-columnar shape, and formed an interface with the model portion. Detachability and an object production accuracy at the interface were good. The obtained object was left to stand still in a thermostat bath of 60 degrees C for 10 minutes and heated. As a result, the support portion was liquefied, and it was possible to easily obtain the model portion. The support portion that had slightly remained on the model portion was removed by application of ultrasonic waves (available form As One Corporation: ASU-6) for 5 minutes in hot water of 60 degrees C. The obtained model portion was not rough on the surface that had contacted the support portion, and it was possible to remove the support portion without residue.

Aspects of the present invention are as follows, for example.

<1> An active-energy-ray-curable composition including:

a monomer (A) having a hydrogen-bonding capacity; and
a compound (B) having a hydrogen-bonding capacity and containing straight-chain alkyl containing 4 or more carbon atoms,

wherein a cured product of the active-energy-ray-curable composition is a solid that exhibits a compressive stress of 2 kPa or higher in response to compression by 1%, and

wherein the active-energy-ray-curable composition is a liquid in an environment of 60 degrees C.

<2> The active-energy-ray-curable composition according to <1>,
wherein the compound (B) having a hydrogen-bonding capacity and containing straight-chain alkyl containing 4 or more carbon atoms is higher alcohol.

<3> The active-energy-ray-curable composition according to <1>,
wherein the compound (B) having a hydrogen-bonding capacity and containing straight-chain alkyl containing 4 or more carbon atoms is diol.

<4> The active-energy-ray-curable composition according to any one of <1> to <3>,
wherein the monomer (A) having a hydrogen-bonding capacity is a water-soluble monofunctional ethylenically unsaturated monomer.

<5> The active-energy-ray-curable composition according to any one of <1> to <4>,
wherein the water-soluble monofunctional ethylenically unsaturated monomer is at least one selected from the group consisting of acryloylmorpholine, hydroxyethyl acrylamide, and diethyl acrylamide.

<6> The active-energy-ray-curable composition according to any one of <1> to <5>,
wherein the compound (B) having a hydrogen-bonding capacity and containing straight-chain alkyl containing 4 or more carbon atoms is compatible with the monomer (A) having a hydrogen-bonding capacity and is a non-reactive compound.

<7> An active-energy-ray-curable composition including:

a polymer (A) having a hydrogen-bonding capacity; and
a compound (B) having a hydrogen-bonding capacity and containing straight-chain alkyl containing 4 or more carbon atoms,

wherein the active-energy-ray-curable composition is a solid that exhibits a compressive stress of 2 kPa or higher in response to compression by 1% in an environment of 25 degrees C, and
wherein the active-energy-ray-curable composition is a liquid having a viscosity of 100 mPa·s or lower in an environment of 60 degrees C.

<8> The active-energy-ray-curable composition according to any one of <1> to <7>, further including a photopolymerization initiator (C).

<9> The active-energy-ray-curable composition according to <8>, wherein the photopolymerization initiator (C) is hydroxycyclohexyl phenyl ketone.

<10> The active-energy-ray-curable composition according to <8> or <9>,
wherein a content of the photopolymerization initiator (C) is from 0.5% by mass through 6% by mass.

<11> The active-energy-ray-curable composition according to any one of <1> to <10>,
wherein a content of the compound (B) having a hydrogen-bonding capacity and containing straight-chain alkyl containing 4 or more carbon atoms is from 20% by mass through 80% by mass.

<12> A three-dimensional object producing method including
using the active-energy-ray-curable composition according to any one of <1> to <11>.

<13> A three-dimensional object producing method including
forming a model portion and a support portion and subsequently removing the support portion, to produce a three-dimensional object,
wherein the three-dimensional object producing method forms the support portion using the active-energy-ray-curable composition according to any one of <1> to <11>, and subsequently removes the support portion.

<14> The three-dimensional object producing method according to <13>,
wherein the removing is removing the support portion by heating the support portion to liquefy the support portion.

<15> The three-dimensional object producing method according to any one of <12> to <14>, further including:

forming a liquid film formed of a liquid containing the active-energy-ray-curable composition according to any one of <1> to <11>; and
curing the liquid film.

<16> The three-dimensional object producing method according to <15>,
wherein the forming a liquid film is performed according to an inkjet method.

<17> A three-dimensional object producing apparatus including:

a liquid film forming unit configured to form a liquid film using the active-energy-ray-curable composition according to any one of <1> to <11> while controlling a coating position and a coating amount of the active-energy-ray-curable composition; and

a unit configured to cure the liquid film.

<18> The three-dimensional object producing apparatus according to <17>, wherein the liquid film forming unit is operated according to an inkjet method.

The active-energy-ray-curable composition according to any one of <1> to <11>, the three-dimensional object producing method according to any one of <12> to <16>, and the three-dimensional object producing apparatus according to <17> or <18> can solve the various problems in the related art and can achieve the object of the present invention.

**Claims**

1. An active-energy-ray-curable composition comprising:

   a monomer (A) having a hydrogen-bonding capacity; and
   a compound (B) that has a hydrogen-bonding capacity and comprises straight-chain alkyl that comprises 4 or more carbon atoms,

   wherein a cured product of the active-energy-ray-curable composition is a solid that exhibits a compressive stress of 2 kPa or higher in response to compression by 1%, and
   wherein the active-energy-ray-curable composition is a liquid in an environment of 60 degrees C.

2. The active-energy-ray-curable composition according to claim 1,
   wherein the compound (B) that has a hydrogen-bonding capacity and comprises straight-chain alkyl that comprises 4 or more carbon atoms comprises higher alcohol.

3. The active-energy-ray-curable composition according to claim 1,
   wherein the compound (B) that has a hydrogen-bonding capacity and comprises straight-chain alkyl that comprises 4 or more carbon atoms comprises diol.

4. The active-energy-ray-curable composition according to any one of claims 1 to 3,
   wherein the monomer (A) having a hydrogen-bonding capacity comprises a water-soluble monofunctional ethylenically unsaturated monomer.

5. The active-energy-ray-curable composition according to any one of claims 1 to 4,
   wherein the compound (B) that has a hydrogen-bonding capacity and comprises straight-chain alkyl that comprises 4 or more carbon atoms is compatible with the monomer (A) having a hydrogen-bonding capacity and is a non-reactive compound.

6. An active-energy-ray-curable composition comprising:

   a polymer (A) having a hydrogen-bonding capacity; and
   a compound (B) that has a hydrogen-bonding capacity and comprises straight-chain alkyl that comprises 4 or more carbon atoms,

   wherein the active-energy-ray-curable composition is a solid that exhibits a compressive stress of 2 kPa or higher in response to compression by 1% in an environment of 25 degrees C, and
   wherein the active-energy-ray-curable composition is a liquid having a viscosity of 100 mPa·s or lower in an environment of 60 degrees C.

7. A three-dimensional object producing method comprising
   using the active-energy-ray-curable composition according to any one of claims 1 to 6.

8. A three-dimensional object producing method comprising
   forming a model portion and a support portion and subsequently removing the support portion, to produce a three-dimensional object,
   wherein the three-dimensional object producing method forms the support portion using the active-energy-ray-curable composition according to any one of claims 1 to 6, and subsequently removes the support portion.

9. The three-dimensional object producing method according to claim 8,
wherein the removing is removing the support portion by heating the support portion to liquefy the support portion.

10. A three-dimensional object producing apparatus comprising:

a liquid film forming unit configured to form a liquid film using the active-energy-ray-curable composition according to any one of claims 1 to 6 while controlling a coating position and a coating amount of the active-energy-ray-curable composition; and
a unit configured to cure the liquid film.

# FIG. 1

# FIG. 2

# FIG. 3

# FIG. 4

**INTERNATIONAL SEARCH REPORT**

| International application No. |
| --- |
| PCT/JP2017/011009 |

| A. CLASSIFICATION OF SUBJECT MATTER |
| --- |
| *C08F2/44*(2006.01)i, *B29C67/00*(2017.01)i, *B33Y10/00*(2015.01)n, *B33Y30/00* (2015.01)n, *B33Y70/00*(2015.01)n |

According to International Patent Classification (IPC) or to both national classification and IPC

| B. FIELDS SEARCHED |
| --- |

Minimum documentation searched (classification system followed by classification symbols)
C08F2/44-2/50, B29C67/00, B33Y10/00, B33Y30/00, B33Y70/00

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| A | JP 2000-212292 A (Sekisui Chemical Co., Ltd.), 02 August 2000 (02.08.2000), claims; paragraphs [0012] to [0017]; examples (Family: none) | 1-6 |
| A | JP 2005-508404 A (3D Systems, Inc.), 31 March 2005 (31.03.2005), claims; examples & US 2003/0092820 A1 claims; examples & WO 2003/029365 A1    & EP 1456307 A1 | 1-10 |

| ☒ Further documents are listed in the continuation of Box C. | ☐ See patent family annex. |
| --- | --- |

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search <br> 02 June 2017 (02.06.17) | Date of mailing of the international search report <br> 13 June 2017 (13.06.17) |
| --- | --- |
| Name and mailing address of the ISA/ <br> Japan Patent Office <br> 3-4-3,Kasumigaseki,Chiyoda-ku, <br> Tokyo 100-8915,Japan | Authorized officer <br><br> Telephone No. |

Form PCT/ISA/210 (second sheet) (January 2015)

**INTERNATIONAL SEARCH REPORT**

| International application No. |
| --- |
| PCT/JP2017/011009 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2000-309703 A  (3D Systems, Inc.), 07 November 2000 (07.11.2000), claims; examples & US 6132665 A claims; examples & EP 1033222 A1 | 1-10 |
| A | JP 2015-183103 A  (JMS GmbH), 22 October 2015 (22.10.2015), claims; paragraph [0034]; examples & US 2017/0001382 A1 claims; paragraph [0062]; examples & WO 2015/144761 A1      & EP 3122537 A1 | 1-10 |
| A | JP 2016-2683 A  (Konica Minolta, Inc.), 12 January 2016 (12.01.2016), claims; paragraphs [0078] to [0082], [0104] (Family: none) | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP T2003535712 W
- JP 2012111226 A
- US 8575258 B
- JP 2015136895 A